# EUROPEAN PATENT APPLICATION

(11) **EP 3 181 038 A1**
(43) Date of publication of application: **21.06.2017**
(21) Application number: 15199761.6
(22) Date of filing: 14.12.2015
(51) Int. Cl.: A61B 5/024

(54) **HEART RATE MEASUREMENT METHOD AND HEART RATE MEASUREMENT DEVICE APPLYING THE SAME**

(71) Applicant: Cheng Uei Precision Industry Co., Ltd., New Taipei City 23680 (TW)
(72) Inventor: Lee, James, 23680 New Taipei City (TW)
(74) Representative: Gee, Steven William

(57) **Abstract**

A heart rate measurement device applies a heart rate measurement method for measuring heart rates of a subject. The heart rate measurement device includes a microprocessor, a photoplethysmography sensing module electrically connected with and controlled by the microprocessor, a triaxial accelerometer sensor electrically connected with the microprocessor, and an analog-to-digital converter. The photoplethysmography sensing module senses biological signals of the subject. The triaxial accelerometer sensor senses whether the subject is in a motion status and sends sensing results to the microprocessor. The analog-to-digital converter is electrically connected with the microprocessor and the photoplethysmography sensing module. The analog-to-digital converter converts the biological signals into digital signals and sends the digital signals to the microprocessor.

## Description

### BACKGROUND OF THE INVENTION

### 1. Field of the Invention

The present invention generally relates to a measurement method and a measurement device, and more particularly to a heart rate measurement method and a heart rate measurement device applying the heart rate measurement method for measuring heart rates.

### 2. The Related Art

A heart rate measurement device is widely used for measuring a heart rate variability and heart rates of a human body. The heart rate measurement device often applies a time domain analysis algorithm to measure the heart rate variability and the heart rates. In the time domain analysis algorithm, a timer axis is acted as a coordinate axis for showing relations of dynamic signals. A time-domain amplifier and a time-domain filter are used in the time domain analysis algorithm for processing the dynamic signals. After the dynamic signals are amplified, the amplified dynamic signals are relatively intuitive and easily recognized. Simultaneously, the time domain analysis algorithm is simpler, so the time domain analysis algorithm has been mainly applied in a heart rate measurement method. The time domain analysis algorithm has characteristics of high speed, high timing and easily satisfying requirements, so the time domain analysis algorithm is applied to measure the heart rates. But a signal quality is requested higher in the time domain analysis algorithm, in a motion status, the heart rates are unable to be measured accurately, less interferences are requested, so the time domain analysis algorithm is limited to be used in a static status.

Subsequently, the heart rates are measured by a frequency domain analysis algorithm on account of the frequency domain analysis algorithm being simpler and being applied conveniently. However, more factors need be considered when a subject is in the motion status that results in a period of time delay in the process of the heart rate measurement device applying the frequency domain analysis algorithm to measure the heart rates.

### SUMMARY OF THE INVENTION

An object of the present invention is to provide a heart rate measurement method and a heart rate measurement device. The heart rate measurement method is applied in the heart rate measurement device for measuring heart rates of a subject. Sense biological signals. Convert the biological signals into digital signals. Execute a time domain analysis algorithm and a frequency domain analysis algorithm simultaneously according to the digital signals to respectively get heart rates of a subject calculated by the time domain analysis algorithm and the heart rates calculated by the frequency domain analysis algorithm. Sense whether the subject is in a motion status, if the subject is in the motion status, the microprocessor chooses the heart rates of the subject calculated by the frequency domain analysis algorithm to be displayed, if the subject is in a non-motion status, execute the next step. Judge whether the heart rates are just started to be measured, if a judging result is that the heart rates are just started to be measured, choose the heart rates calculated by the time domain analysis algorithm to be displayed, if the judging result is unstarted to be measured, execute the next step. Compare the heart rates calculated by the time domain analysis algorithm with the heart rates calculated by the frequency domain analysis algorithm to judge whether the heart rates calculated by the time domain analysis algorithm are close to the heart rates calculated by the frequency domain analysis algorithm, if the heart rates calculated by the time domain analysis algorithm are close to the heart rates calculated by the frequency domain analysis algorithm, choose the heart rates of the subject calculated by the time domain analysis algorithm to be displayed, if the heart rates calculated by the time domain analysis algorithm are without being close to the heart rates calculated by the frequency domain analysis algorithm, choose the heart rates of the subject calculated by the frequency domain analysis algorithm to be displayed.

The heart rate measurement device includes a microprocessor, a photoplethysmography sensing module electrically connected with and controlled by the microprocessor, a triaxial accelerometer sensor electrically connected with the microprocessor, and an analog-to-digital converter. The photoplethysmography sensing module senses biological signals of the subject. The triaxial accelerometer sensor senses whether the subject is in a motion status and sends sensing results to the microprocessor. The analog-to-digital converter is electrically connected with the microprocessor and the photoplethysmography sensing module. The analog-to-digital converter converts the biological signals into digital signals and sends the digital signals to the microprocessor.

As described above, the heart rate measurement device applies the heart rate measurement method to make the microprocessor choose the heart rates calculated by the time domain analysis algorithm to be displayed on a display terminal in the non-motion status, and the heart rate measurement device applies the heart rate measurement method to make the microprocessor choose the heart rates of the subject calculated by the frequency domain analysis algorithm to be displayed on a display terminal in the motion status and a buffering period, so that the heart rates of the subject measured by the heart rate measurement device is ensured to be measured fast and accurately in the non-motion status, and in the meanwhile, the heart rates of the subject measured by the heart rate measurement device is ensured to be measured accurately in the motion status and the buffering period.

### BRIEF DESCRIPTION OF THE DRAWINGS

The present invention will be apparent to those skilled in the art by reading the following description, with reference to the attached drawings, in which:
FIG. 1 is a flow chart of a heart rate measurement method in accordance with the present invention; and
FIG. 2 is a functional block diagram of a heart rate measurement device in accordance with the present invention, wherein the heart rate measurement method is applied in the heart rate measurement device.

### DETAILED DESCRIPTION OF THE PREFERRED EMBODIMENT

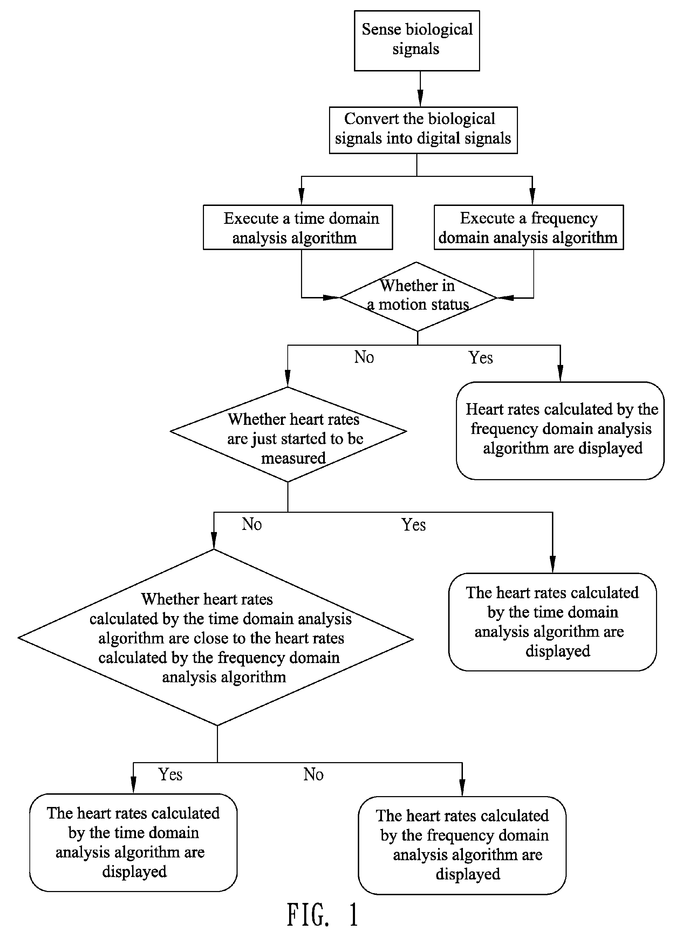

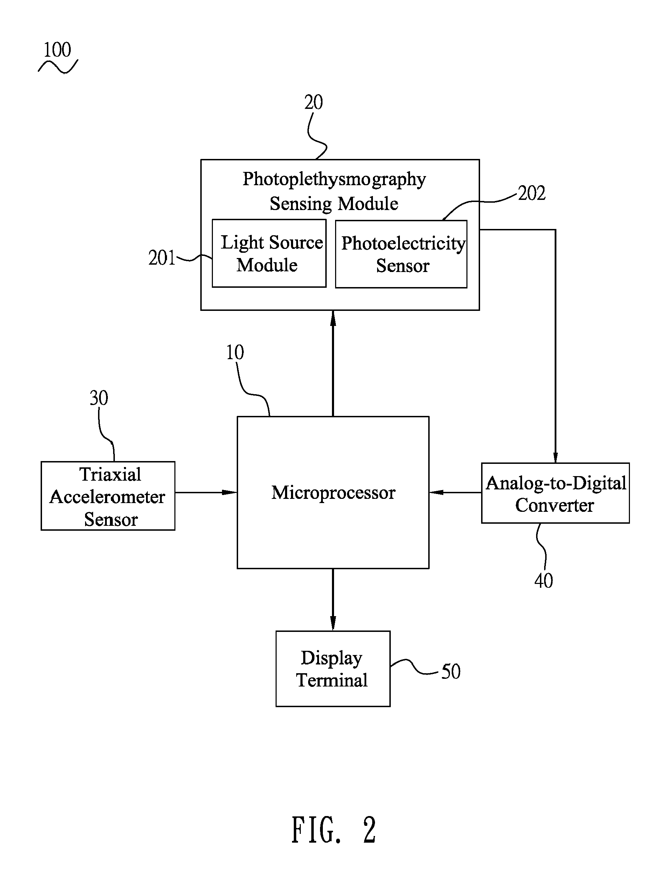

With reference to FIG. 1 and FIG. 2, a heart rate measurement device 100 in accordance with the present invention is shown. The heart rate measurement device 100 applies a heart rate measurement method for measuring heart rates of a subject. The heart rate measurement device 100 includes a microprocessor 10, a photoplethysmography sensing module 20, a triaxial accelerometer sensor 30, an analog-to-digital converter 40 and a display terminal 50.

The photoplethysmography sensing module 20 is electrically connected with and controlled by the microprocessor 10. The photoplethysmography sensing module 20 senses biological signals of the subject. The photoplethysmography sensing module 20 includes a light source module 201 and a photoelectricity sensor 202. The microprocessor 10 controls the light source module 201 to send light beams to skin of the subject. The photoelectricity sensor 202 senses reflected light beams reflected by the skin of the subject and outputs the biological signals to the analog-to-digital converter 40. The analog-to-digital converter 40 is electrically connected with the microprocessor 10 and the photoplethysmography sensing module 20. The analog-to-digital converter 40 converts the biological signals into digital signals and sends the digital signals to the microprocessor 10. The microprocessor 10 receives the digital signals.

The triaxial accelerometer sensor 30 is electrically connected with the microprocessor 10. The triaxial accelerometer sensor 30 senses whether the subject is in a motion status and sends sensing results to the microprocessor 10. The microprocessor 10 is capable of executing a time domain analysis algorithm and a frequency domain analysis algorithm simultaneously according to the received digital signals to respectively get the heart rates of the subject calculated by the time domain analysis algorithm and the heart rates of the subject calculated by the frequency domain analysis algorithm. The display terminal 50 is electrically connected with the microprocessor 10. The heart rates got by the microprocessor 10 are displayed on the display terminal 50. The microprocessor 10 chooses the heart rates calculated by the time domain analysis algorithm or the heart rates calculated by the frequency domain analysis algorithm to be displayed on the display terminal 50 according to whether the subject is in the motion status.

The light source module 201 is an infrared emitting diode. The biological signals are photoplethysmography (PPG) signals. When the heart rate measurement device 100 is turned on to be in an initial status, a normal heart rate will be instantly displayed on the display terminal 50. The display terminal 50 is a displayer of the heart rate measurement device 100, a cell phone or other intelligent devices. The display terminal 50 is without being limited to be a visual display unit. The display terminal 50 also can be an audio display device, etc. Sensing the biological signals of the subject is without being limited to apply the photoplethysmography sensing module 20.

Specific steps of the heart rate measurement method applied in the heart rate measurement device 100 are described as follows.

The photoplethysmography sensing module 20 senses the biological signals and transmits the biological signals to the analog-to-digital converter 40.

The analog-to-digital converter 40 receives the biological signals and converts the biological signals into the digital signals, and sends the digital signals to the microprocessor 10.

The microprocessor 10 executes the time domain analysis algorithm and the frequency domain analysis algorithm simultaneously according to the digital signals to respectively get the heart rates of the subject calculated by the time domain analysis algorithm and the heart rates calculated by the frequency domain analysis algorithm.

The triaxial accelerometer sensor 30 senses whether the subject is in the motion status and sends the sensing results to the microprocessor 10. If the subject is in the motion status, the microprocessor 10 chooses the heart rates of the subject calculated by the frequency domain analysis algorithm to be displayed on the display terminal 50; if the subject is in the non-motion status, execute the next step.

The microprocessor 10 judges whether the heart rates are just started to be measured, if a judging result is that the heart rates are just started to be measured, the microprocessor 10 chooses the heart rates calculated by the time domain analysis algorithm to be displayed on the display terminal 50; if the judging result is unstarted to be measured, execute the next step.

The microprocessor 10 compares the heart rates calculated by the time domain analysis algorithm with the heart rates calculated by the frequency domain analysis algorithm to judge whether the heart rates calculated by the time domain analysis algorithm are close to the heart rates calculated by the frequency domain analysis algorithm. If the heart rates calculated by the time domain analysis algorithm are close to the heart rates calculated by the frequency domain analysis algorithm, the microprocessor 10 chooses the heart rates of the subject calculated by the time domain analysis algorithm to be displayed on the display terminal 50, if the heart rates calculated by the time domain analysis algorithm are without being close to the heart rates calculated by the frequency domain analysis algorithm, the microprocessor 10 chooses the heart rates of the subject calculated by the frequency domain analysis algorithm to be displayed on the display terminal 50.

In a general condition, when the heart rates calculated by the time domain analysis algorithm are without being close to the heart rates calculated by the frequency domain analysis, the subject is in a buffering period after the motion is stopped.

As described above, the heart rate measurement device 100 applies the heart rate measurement method to make the microprocessor 10 choose the heart rates calculated by the time domain analysis algorithm to be displayed on the display terminal 50 in the non-motion status, and the heart rate measurement device 100 applies the heart rate measurement method to make the microprocessor 10 choose the heart rates of the subject calculated by the frequency domain analysis algorithm to be displayed on the display terminal 50 in the motion status and the buffering period, so that the heart rates of the subject measured by the heart rate measurement device 100 is ensured to be measured fast and accurately in the non-motion status, and in the meanwhile, the heart rates of the subject measured by the heart rate measurement device 100 is ensured to be measured accurately in the motion status and the buffering period.

## Claims

1. A heart rate measurement method applied in a heart rate measurement device for measuring heart rates of a subject, comprising steps of:
sensing biological signals;
converting the biological signals into digital signals;
executing a time domain analysis algorithm and a frequency domain analysis algorithm simultaneously according to the digital signals to respectively get the heart rates of a subject calculated by the time domain analysis algorithm and the heart rates calculated by the frequency domain analysis algorithm;
sensing whether the subject is in a motion status, if the subject is in the motion status, the microprocessor choosing the heart rates of the subject calculated by the frequency domain analysis algorithm to be displayed, if the subject is in a non-motion status, executing the next step;
judging whether the heart rates are just started to be measured, if a judging result is that the heart rates are just started to be measured, choosing the heart rates calculated by the time domain analysis algorithm to be displayed, if the judging result is unstarted to be measured, executing the next step; and
comparing the heart rates calculated by the time domain analysis algorithm with the heart rates calculated by the frequency domain analysis algorithm to judge whether the heart rates calculated by the time domain analysis algorithm are close to the heart rates calculated by the frequency domain analysis algorithm, if the heart rates calculated by the time domain analysis algorithm are close to the heart rates calculated by the frequency domain analysis algorithm, choosing the heart rates of the subject calculated by the time domain analysis algorithm to be displayed, if the heart rates calculated by the time domain analysis algorithm are without being close to the heart rates calculated by the frequency domain analysis algorithm, choosing the heart rates of the subject calculated by the frequency domain analysis algorithm to be displayed.

2. The heart rate measurement method as claimed in claim 1, wherein the heart rate measurement device includes a microprocessor, a photoplethysmography sensing module electrically connected with the microprocessor, an analog-to-digital converter electrically connected with the photoplethysmography sensing module and the microprocessor, the photoplethysmography sensing module senses the biological signals and transmits the biological signals to the analog-to-digital converter, the analog-to-digital converter receives the biological signals and converts the biological signals into the digital signals, and sends the digital signals to the microprocessor.

3. The heart rate measurement method as claimed in claim 2, wherein the microprocessor executes the time domain analysis algorithm and the frequency domain analysis algorithm simultaneously according to the digital signals.

4. The heart rate measurement method as claimed in claim 2, wherein the heart rate measurement device further includes a triaxial accelerometer sensor electrically connected with the microprocessor, the triaxial accelerometer sensor senses whether the subject is in the motion status and sends sensing results to the microprocessor, if the subject is in the motion status, the microprocessor chooses the heart rates of the subject calculated by the frequency domain analysis algorithm to be displayed.

5. The heart rate measurement method as claimed in claim 2, wherein the heart rate measurement device further includes a display terminal electrically connected with the microprocessor, the heart rates got by the microprocessor are displayed on the display terminal.

6. The heart rate measurement method as claimed in claim 1, wherein the biological signals are photoplethysmography signals.

7. A heart rate measurement device, comprising:
a microprocessor;
a photoplethysmography sensing module electrically connected with and controlled by the microprocessor, the photoplethysmography sensing module sensing biological signals of the subject;
a triaxial accelerometer sensor electrically connected with the microprocessor, the triaxial accelerometer sensor sensing whether the subject is in a motion status and sending sensing results to the microprocessor; and
an analog-to-digital converter electrically connected with the microprocessor and the photoplethysmography sensing module, the analog-to-digital converter converting the biological signals into digital signals and sending the digital signals to the microprocessor.

8. The heart rate measurement device as claimed in claim 7, wherein the photoplethysmography sensing module includes a light source module and a photoelectricity sensor, the microprocessor controls the light source module to send light beams to skin of the subject, the photoelectricity sensor senses reflected light beams reflected by the skin of the subject and outputs the biological signals to the analog-to-digital converter.

9. The heart rate measurement device as claimed in claim 8, wherein the light source module is an infrared emitting diode.

10. The heart rate measurement device as claimed in claim 7, further comprising a display terminal electrically connected with the microprocessor, heart rates got by the microprocessor being displayed on the display terminal.

11. The heart rate measurement device as claimed in claim 7, wherein the biological signals are photoplethysmography signals.
